# EUROPÄISCHE PATENTSCHRIFT

(11) **EP 0 517 008 B1**
(45) Veröffentlichungstag und Bekanntmachung des Hinweises auf die Patenterteilung: **29.11.1995**
(21) Anmeldenummer: 92108003.2
(22) Anmeldetag: 12.05.1992
(51) Int. Cl.: B01L 3/00, B65D 90/00, B65D 85/30, B65F 1/00, B01L 9/02

(54) **Vorrichtung zum Entsorgen von Laborabfällen**
Apparatus for the disposal of laboratory waste
Dispositif pour l'enlèvement de déchets de laboratoire

(30) Priorität: 04.06.1991 DE 4118314
(43) Veröffentlichungstag der Anmeldung: 09.12.1992
(73) Patentinhaber: Waldner Laboreinrichtungen GmbH & Co., D-88231 Wangen (DE)
(72) Erfinder: Kreuzer, Konrad, W-8969 Dietmannsried (DE); Knop, Winfried, W-7988 Wangen (DE); Reichard, Dietrich, W-5308 Rheinbach (DE)
(74) Vertreter: WILHELMS, KILIAN & PARTNER Patentanwälte

(56) Entgegenhaltungen:
- EP-A- 0 072 208
- EP-A- 0 098 672
- WO-A-90/08715
- DE-U- 8 631 104
- US-A- 2 755 971
- US-A- 2 934 390
- US-A- 4 249 785
- DATABASE WPIL Section Ch, Week 8242, Derwent Publications Ltd., London, GB;Class J04, AN 82-89178E/42 & SE-B-444 271 (G.C. STRANDLUND et al), 7. April 1986
- RESEARCH DISCLOSURE Nr. 318, Oktober 1990, HAVANT GB Seite 822 , XP148666'Laboratory barrel waste containment'

## Beschreibung

Die Erfindung betrifft eine Vorrichtung zum Entsorgen von Laborabfällen.

In Laboratorien fallen verschiedenste Chemieabfälle, wie Lösungsmittel und lösungsmittelhaltige und brennbare Stoffe, anorganische wässrige Lösungen und Feststoffe, insbesondere chemisch benetzte Feststoffe an, die aus Gründen der Sicherheit entsorgt und ggf. zwischengelagert werden müssen.

In Laboratorien werden bisher die Nutzchemikalien für die tägliche Arbeit, insbesondere flüssige Chemikalien, d.h., hauptsächlich Lösungsmittel aller Art, sowie Säuren, Laugen und Salze bereitgestellt. Dafür stehen spezielle Schränke zur Verfügung, für die einschlägige Normen, beispielsweise DIN-Richtlinien und Laborrichtlinien gelten.

Für die entstehenden Laborabfallstoffe gibt es jedoch bisher kein einheitliches Entsorgungskonzept, sondern lediglich Partiallösungen.

So gibt es in Labors in der Regel Behälter aus Glas, Kunststoff, Stahl oder Kunststoffbehälter mit Stahlummantelung zur Aufnahme von Laborabfällen, die Handhabung dieser Behälter, d.h. insbesondere das Füllen, Transportieren, Entleeren und Lagern dieser Behälter, bleibt jedoch weitgehend den Laborbetreibern überlassen.

D.h., daß in der Praxis diese Entsorgungsbehälter entweder in einen Abzug oder in einen belüfteten Unterschrank gestellt werden. Es kommt allerdings auch vor, daß diese Entsorgungsbehälter offen auf einem Tisch oder auf dem Fußboden stehen. Mit dieser Art der Handhabung der Entsorgungsbehälter sind erhebliche Gefahren für das Personal verbunden, da beim Befüllen Dämpfe entstehen können oder während der Lagerung entstehende Dämpfe dem Behälter entweichen können, die das Laborpersonal gefährden, oder die Behälter umfallen können.

Wenn die Behälter in Abzügen aufgestellt werden, ist diese Gefährdung zwar nicht gegeben, diese Lösung ist jedoch insofern nachteilig, als Abzüge einen wertvollen Arbeitsraum darstellen, der nicht durch die Lagerung von Chemieabfällen verschwendet werden sollte.

Auf dem Radionuklidbereich wird von einer weiteren Möglichkeit der Aufbewahrung von flüssigen Abfällen mittels sogenannter fahrbarer Flaschenwagen Gebrauch gemacht. Dabei wird in der Tischplatte eines Abzuges eine Öffnung vorgesehen und werden die aufzubewahrenden flüssigen Abfällen von dort über eine Schlauchverbindung in einen Behälter geleitet, der in einem fahrbaren Wagen unterhalb des Abzuges steht. Die beim Füllen des Behälters möglicherweise entstehenden Dämpfe werden in den Abzug geleitet und dort mit der Abzugsluft abgeführt. Diese Art der Entsorgung ist zwar gegenüber der freien Lagerung von Entsorgungsbehältern eindeutig vorteilhaft, sie ist jedoch weiterhin mit gewissen Nachteilen verbunden. Einmal erfolgt die Belüftung der Behälter über den Abzug. Das hat zur Folge, daß der Abzug, der ein vielfach höheres Luftvolumen als ein abgesaugter Behälter benötigt, ständig in Betrieb gehalten werden muß, um einen Austritt von Schadstoffen zu vermeiden. Der Behälter ist darüberhinaus uneinsichtig angeordnet, so daß für die Messung des Füllstandes eine aufwendige elektrische Wiegevorrichtung notwendig ist. Es ist schließlich auch nicht auszuschließen, daß Dämpfe aus der Leitungsverbindung zwischen dem Abzug und dem Behälter austreten, wenn diese nicht vorschriftsmäßig angebracht ist, was schlecht zu kontrollieren ist.

Aus dem DE-GM 86 31 104 ist bereits ein Einfülltrichter zum Befüllen eines Behälters mit brennbaren Flüssigkeiten bekannt, der mit einer Überfüllsicherung versehen ist. Dieser Einfülltrichter wird auf den Behälter geschraubt und gewährleistet ein sicheres Füllen des Behälters, wobei gleichzeitig eine Flammensperre vorgesehen ist. Ein derartiger Einfülltrichter eignet sich jedoch nicht für andere Laborabfälle, wie beispielsweise wässrige anorganische Lösungen, da einmal das Material des Einfülltrichters nicht säurebeständig ist, und zum anderen der Einfülltrichter bei geschlossenem Deckel keine Druckausgleichsmöglichkeit für die bei chemischen Reaktionen von wässrigen Lösungen entstehenden Volumenänderungen bietet.

Die der Erfindung zugrunde liegende Aufgabe besteht daher darin, eine Vorrichtung zum Entsorgen von Laborabfällen zu schaffen, die eine einfache und dennoch sichere Entsorgung verschiedenartigster Laborabfälle, wie Lösungsmittel und lösungsmittelhaltiger brennbarer Stoffe, anorganischer wässriger Lösungen und von Feststoffen insbesondere chemisch benetzten Feststoffen erlaubt.

Zur Lösung dieser Aufgabe ist die erfindungsgemäße Vorrichtung durch die Ausbildung gekennzeichnet, die im Kennzeichen des Patentanspruchs 1 angegeben ist.

Die erfindungsgemäße Vorrichtung ist somit so ausgebildet, daß lösungsmittelhaltige und brennbare Stoffe, anorganische wässrige Lösungen oder Feststoffe, wie beispielsweise chemisch benetzte Feststoffe, entsorgt werden können, indem dementsprechende Behälter vorgesehen werden.

Zur Verwendung der erfindungsgemäßen Vorrichtung werden daher die Laborabfälle in diese drei Stoffgruppen zunächst unterteilt und wird für jede Abfallart ein entsprechender Behälter vorgesehen und in einer speziell dafür geeigneten Weise gefüllt und gelagert. Der Behälter kann dann so ausgebildet sein, daß er gegenüber dem Füllgut beständig ist.

Die Auftrennung der Laborabfälle in die o.a. Stoffe ist dadurch bedingt, daß jede Stoffart speziell in anderer Weise behandelt werden muß.

Besonders bevorzugte Weiterbildungen und Ausgestaltungen der erfindungsgemäßen Vorrichtung sind Gegenstand der Patentansprüche 2 bis 13.

D.h. im einzelnen, daß bei lösungsmittelhaltigen oder brennbaren Stoffen das Hauptaugenmerk darin liegt, daß diese Stoffe einen ständigen Dampfdruck haben, sowie brennbar sind und in geeigneter Konzentration mit der Luft zur Bildung explosiver Gemische neigen. Dabei ist gleichzeitig zu berücksichtigen, daß der entsprechende Behälter lösungsmittelbeständig sein muß, d.h., aus einem geeigneten Kunststoffbehälter, der von einem Stahlbehälter umschlossen ist, oder direkt aus einem Metall bestehen muß. Die dabei vorgesehene Einfüllvorrichtung muß einerseits geerdet und leitfähig sein, und andererseits als Flammensperre dienen. Aufgrund des Dampfdruckes muß die Einfüllvorrichtung weiterhin so ausgebildet sein, daß sie nach ihrem Gebrauch verschließbar ist. Diese Ausbildung ist Gegenstand der Patentansprüche 6 und 7.

Bei organischen wässrigen Lösungen, unter denen Gemische aus Säuren, Laugen oder Salzen zu verstehen sind, ist zum einen die Korrosionsgefahr zu berücksichtigen. Das bedeutet, daß der Behälter kein Metallbehälter sein darf, sondern aus einem chemischbeständigen Kunststoff bestehen muß. Während der Zwischenlagerung kann es zum anderen zu chemischen Reaktionen zwischen den einzelnen Stoffen kommen. Beispielsweise können Neutralisationsreaktionen zwischen Säuren und Laugen auftreten, die zu einer Erwärmung und damit einer Volumenausdehnung führen. Weiterhin kann es zu Verdrängungsreaktionen zwischen verschieden starken Säuren kommen, so daß die starke Säure, beispielsweise eine Zitronensäure, die schwache Säure, wie beispielsweise eine Kohlensäure, austreibt. Während der Lagerung ist daher mit einem Austrag von Gasen und damit einer Volumenvergrößerung zu rechnen.

Das heißt, daß bei anorganischen wässrigen Lösungen der Behälter auch mit einer geeigneten Entlüftung versehen sein muß, die nicht verschlossen werden darf. Im Gegensatz zu Lösungsmitteldämpfen, die schwerer als Luft sind, sind die Dämpfe von anorganischen wässrigen Lösungen teilweise wesentlich leichter als Luft. Beim Einfüllen ist es daher vorteilhaft, wenn die Entlüftung möglichst tief angesiedelt ist, um ein leichtes Absaugen der entstehenden Dämpfe zu gewährleisten. Um das zu erzielen, kann die erfindungsgemäße Vorrichtung so ausgebildet sein, wie es in den Patentansprüchen 8 bis 13 angegeben ist.

Zur Entsorgung von anorganischen wässrigen Lösungen wird dabei der Einfülltrichter auf den Entsorgungsbehälter geschraubt, der mit einem Schwimmer, einem Füllstandsanzeiger und einer Entlüftung versehen ist. Die Dämpfe, die beim Einfüllen aus dem Behälter getrieben werden, gehen über die Entlüftung nach außen, wobei diese Entlüftung vorzugsweise im Überwurfdeckel des Einfülltrichters integriert ist. Der Überwurfdeckel ist an den Trichterhals geschweißt und wird auf den Entsorgungsbehälter geschraubt. Dadurch entweichen die Dämpfe an einer relativ tiefen Stelle, so daß sie gut abgesaugt werden können.

Der Füllstand des Behälters wird durch den mechanischen Peilstab angezeigt, der mit dem Füllstand ansteigt. Mit einem derartigen Einfülltrichter für wässrigen Lösungsmittel wird eine sicheres Entweichen der Dämpfe im Behälter gewährleistet und ist eine ständig geöffnete Überfüllöffnung sichergestellt, so daß aufgrund von chemischen Reaktionen entstehende Überdrücke gefahrenlos abgebaut werden können.

Wenn bei der erfindungsgemäßen Vorrichtung die Oberkante des Innenbehälters, in dem der Entsorgungsbehälter angeordnet ist, höher als die Oberkante des Einfülltrichters liegt, können die beim Einfüllen entstehenden Dämpfe nicht in den Laborraum gelangen. Da der Innenbehälter abgesaugt wird, ist sicher gewährleistet, daß die entstehenden Dämpfe zuverlässig abgeführt werden. Da zum Absaugen hierbei große Luftmengen benötigt werden, die bei einem geschlossenen Entsorgungsbehälter nicht notwendig sind, ist eine Ausbildung bevorzugt, bei der dann, wenn die Behälter herausgezogen werden, durch eine mechanische Vorrichtung die Luftmenge erhöht und beim Hereinschieben wieder gedrosselt wird.

Wenn der Behälter auf einer herausnehmbaren Auffangwanne oder Einsatzschale insbesondere auf einem darin angeordneten Gitterrost steht, dann kann bei einem fehlerhaften Füllen des Behälters dieser entnommen werden, wobei die übergelaufene Flüssigkeit sich in der Einsatzschale sammelt. Sie kann herausgenommen und gereinigt werden. Die Einsatzschale besteht aus einem dementsprechend beständigen Material.

Da die Behälter ein Gewicht von bis zu 30 kg haben können und zum sicheren Absaugen ein allseits geschlossener Innenbehälter notwendig ist, ist es vorteilhaft, wenn dieser eine aufklappbare Front, vorzugsweise ein aufklappbare Vorderfront hat. Nach dem Aufklappen der Vorderfront kann der Behälter höhengleich auf einen dafür geeigneten fahrbaren Wagen ohne Anheben gezogen werden.

Bei der Entsorgung von Feststoffen, insbesonder chemisch benetzten Feststoffen, worunter beispielsweise Filter, wie benetzte Filterpapiere und verschiedene ähnliche Materialien zu verstehen sind, wird vorzugsweise ein Weithalsbehälter aus einem geeigneten Kunststoff- oder Metallmaterial verwandt.

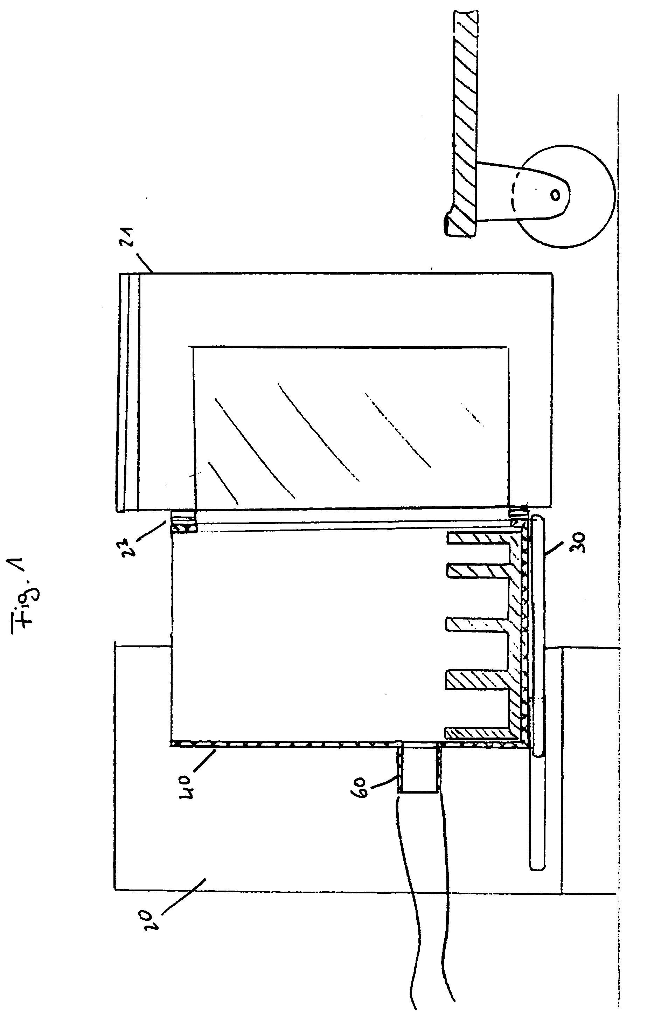

Im folgenden wird anhand der zugehörigen Zeichnung ein besonders bevorzugtes Ausführungsbeispiel der Erfindung näher beschrieben. Es zeigen
Fig. 1 eine Schnittansicht des Ausführungsbeispiels der erfindungsgemäßen Vorrichtung im ausgezogenen Zustand,
Fig. 2 eine perspektivische Darstellung des Grundaufbaus, wobei der Außenkorpus und die Vorderfront gestrichelt dargestellt sind,
Fig. 3 eine perspektivische Darstellung des Innenbehälters mit integrierter Abluftregelung und nebenstehenden Einschubwanne und
Fig. 4 eine Schnittansicht eines Ausführungsbeispiels des Einfülltrichters zur Entsorgung von anorganischen wässrigen Lösungen.

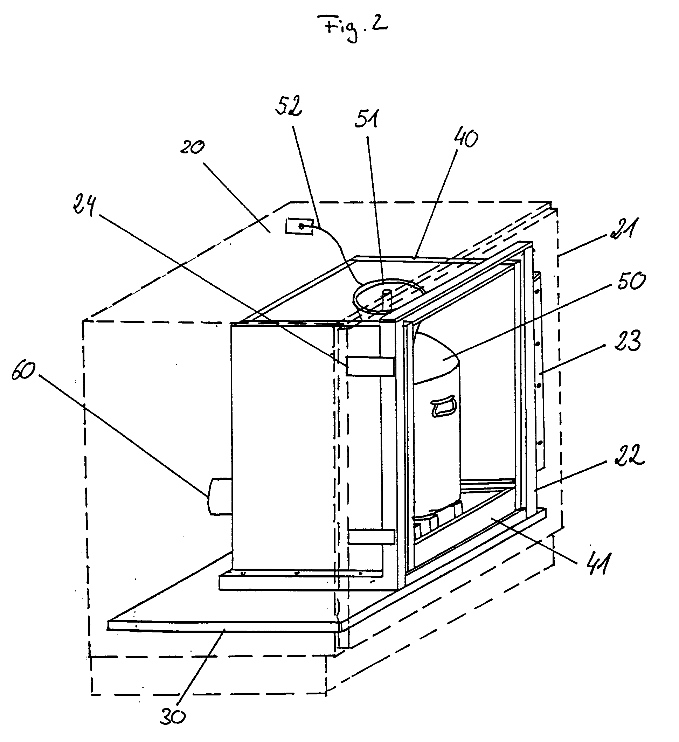

Das in Fig. 2 dargestellte Ausführungsbeispiel der erfindungsgemäßen Vorrichtung umfaßt einen Außenkorpus 20, in den verschiedene Einsätze zur Entsorgung der verschiedenen Abfallstoffe und Chemikalien eingebaut werden können. Der Außenkorpus 20 besteht aus zwei Seitenteilen, einem oberen und einem unteren Boden, sowie einer Rückwand. Die Vorderfront 21 ist an einem Stahlgestell 22 befestigt. Diese Front 21 kann über ein Scharnier 23 weggeschwenkt werden, nachdem ein ausziehbarer Fachboden 30 herausgezogen ist. Eine Verriegelung der Tür des Innenbehälters oder Einstellbehälters 40 mit dem Stahlgestell 22 erfolgt über einen Verschluß 24. Das Stahlgestell 22 wird fest auf dem Boden verschraubt und bildet die seitliche Halterung des Einstellbehälters 40. Der Einstellbehälter 40 ist an drei Seiten geschlossen. Seine vierte Seite oder Tür ist fest mit der Vorderfront 21 verbunden und schwenkt beim Öffnen zum Entleeren mit der Vorderfront 21 weg. Am Boden des Innenbehälters 40 ist eine Einsatzschale 41 angeordnet, die als Wanne geformt ist, so daß sie Flüssigkeiten von dem eigentlichen Aufnahmebehälter 50 aufnehmen kann, der darüber angeordnet ist. Die Einsatzschale 41 weist insbesondere einen Gitterrost oder Rippen auf, auf dem oder auf denen der Aufnahmebehälter 50 steht. Auf den Aufnahmebehälter 50 ist ein Einfülltrichter 51 geschraubt.

Bei der Entsorgung von Lösungsmitteln oder lösungsmittelhaltigen und brennbaren Abfällen ist der Einfülltrichter 51 mit einem Erdungs- oder Potentialausgleichskabel 52 ständig verbunden. Beim Einfüllen der zu entsorgenden Flüssigkeit in den Einfülltrichter 51 werden die Dämpfe nach unten durch einen Einfüllstutzen 60 am Innenbehälter 40 abgesaugt. Die Außenwände des Innenbehälters sollten dabei höher als der obere Rand des Einfülltrichters sein.

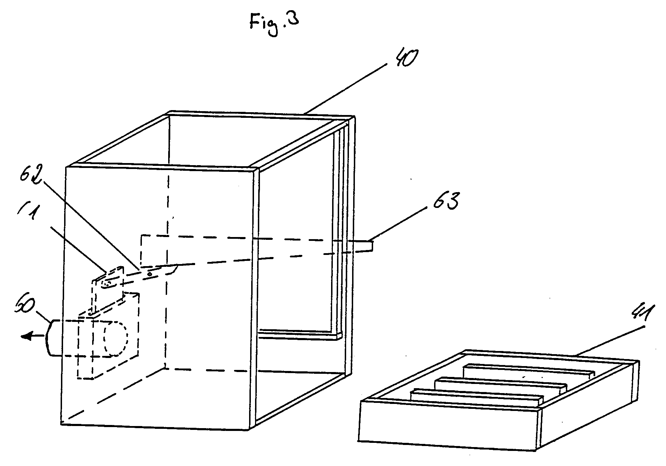

In Fig. 3 ist die Regelung der Abluft dargestellt. Ein Endrohr 60 wird mit dem bauseitigen Abluftsystem fest verbunden. Ein Schieber 61 wird über ein Gestänge 62 und eine Laufleiste 63 bewegt. Im ausgezogenen Zustand des Fachbodens 30 öffnet der Schieber 61 den gesamten Querschnitt. Wird der Fachboden 30 eingeschoben, so verringert sich der Querschnitt bis auf 20 % seiner normalen Größe.

Der Grund für diese Regelung besteht darin, daß im herausgezogenen Zustand die Flüssigkeiten eingefüllt werden, so daß eine hohe Luftmenge zum Absaugen zur Verfügung steht, die im eingeschobenen Zustand nicht mehr benötigt wird.

Je nach der Art der zu entsorgenden Stoffe werden entsprechende Aufnahmebehälter 30 verwandt. Für Lösungsmittel oder löstungsmittelhaltige Stoffe werden Kombi-Behälter mit äußerem Stahlbehälter und innerem Kunststoff-Enghalsbehälter mit Metalltrichter vorgesehen, für wässrige anorganische Lösungen werden Kunststoffbehälter mit Kunststofftrichter verwandt und für Feststoffe, insbesondere chemisch benetzte Feststoffe werden Kombi-Behälter mit äußerem Stahlbehälter und innerem Kunststoff-Weithalsbehälter vorgesehen.

Der Einfülltrichter 24, der bei der Entsorgung von wässrigen anorganischen Lösungen verwandt wird, ist vorzugsweise so ausgebildet, wie es in Fig. 3 dargestellt ist.

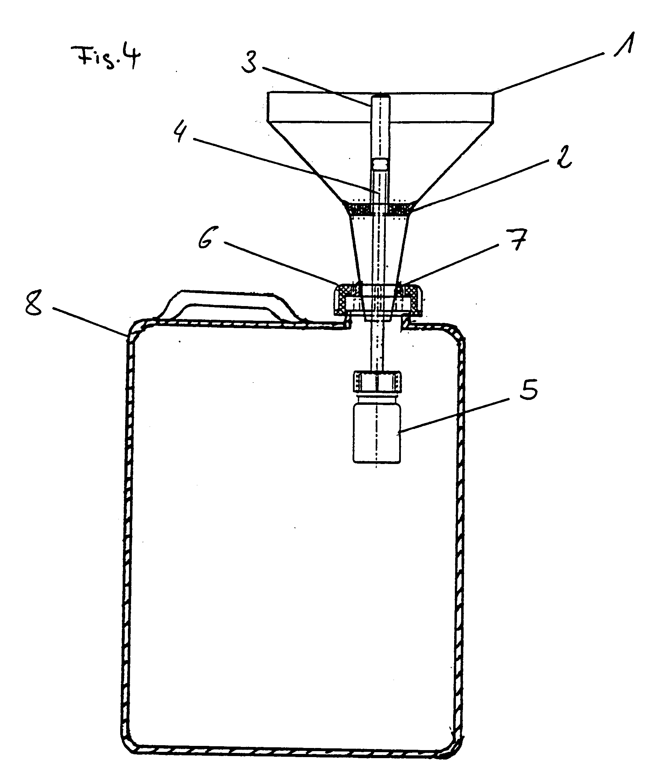

Der in Fig. 4 dargestellte Einfülltrichter besteht aus einem Weithalstrichter 1 mit integrierter Füllstandsanzeige und einem Sieb 2. Die optische Füllstandsanzeige besteht aus einem glasklaren Standrohr 3, welches im Sieb 2 fest angebracht ist. In diesem Standrohr erscheint bei einer entsprechenden Füllhöhe des Behälters 8 ein Stab 4, der durch einen Schwimmer 5 nach oben verschoben wird. Der Trichter selbst ist fest mit einer Überwurfmutter 6 verschweißt, die einen Kranz mit Entlüftungsbohrungen 7 aufweist.

Der Einfülltrichter wird auf den Aufnahmebehälter 8 geschraubt und dient zum sicheren Befüllen des Behälters. Beim Einfüllen einer Flüssigkeit in den Trichter bleiben grobe Verunreinigungen im Sieb 2 hängen. Die beim Befüllen des Behälters verdrängten Dämpfe und Gase entweichen durch die Entlüftungsbohrungen 7 aus dem Behälter 8 und können dort abgesaugt werden.

Entstehen bei der Lagerung im Inneren des Behälters 8 chemische Zersetzungsprodukte oder treten Volumenänderungen durch chemische Reaktionen auf, so können die Dämpfe ebenfalls durch die Entlüftungsbohrungen 7 entweichen, ohne daß der Behälter aufgebläht oder der Innendruck erhöht wird.

Die gesamte Vorrichtung kann vorzugsweise unter einem Tisch eingebaut werden, z.B. als Unterbau für einen Gasabzug. Es ist aber auch ein Einbau in Hochschränke möglich. Dabei kann der untere Teil des Hochschrankes für die Entsorgungsvorrichtung vorgesehen werden, während der obere Teil des Hochschrankes für die Versorgung mit Laborchemiekalien bestimmt ist.

## Patentansprüche

1. Vorrichtung zum Entsorgen von Laborabfällen, die aufweist
- einen Außenkorpus (20), der an einer Seite geöffnet werden kann und im Inneren einen aus der geöffneten Seite herausziehbaren Fachboden (30) aufweist,
- einen an der Oberseite offenen Innenbehälter (40), der abnehmbar auf dem Fachboden des Außenkorpus (20) angeordnet und mit einem regelbaren Abluftsystem versehen ist, das an eine Entlüftungsanlage angeschlossen werden kann,
- eine Einsatzschale (41), die auf dem Boden des Innenbehälters (40) angeordnet ist, und
- einen Aufnahmebehälter (50) für die Laborabfälle, der durch die Einsatzschale (41) gehalten ist.

2. Vorrichtung nach Anspruch 1, dadurch gekennzeichnet, daß eine Seite des Innenbehälters (40) geöffnet werden kann und mit der Seite des Außenkorpus (20), der geöffnet werden kann, in Form eines Frontdoppels in Verbindung steht, wobei beide Teile zum Entleeren nach dem Herausziehen des Fachbodens wegdrehbar sind.

3. Vorrichtung nach Anspruch 1 oder 2, dadurch gekennzeichnet, daß in der Einsatzschale (41) ein Gitterrost angeordnet ist, auf dem der Aufnahmebehälter (50) steht.

4. Vorrichtung nach Anspruch 3, gekennzeichnet durch einen Transportwagen, dessen Transportfläche unter der Höhe des Gitterrostes in der Einsatzschale (41) liegt.

5. Vorrichtung nach einem der vorhergehenden Ansprüche, gekennzeichnet durch eine Einrichtung (60 - 63), die einen Entlüftungsstutzen (60) öffnet, wenn der Fachboden im Außenkorpus (20) herausgezogen wird, und beim Zurückschieben des Fachbodens die Querschnittsfläche des Entlüftungsstutzens (60) verringert.

6. Vorrichtung nach einem der vorhergehenden Ansprüche zur Entsorgung von Lösungsmitteln und lösungsmittelhaltigen brennbaren Stoffen, dadurch gekennzeichnet, daß der Aufnahmebehälter (50) aus einem von einem Stahlmantel umgebenen Kunststoffbehälter besteht, auf den ein metallischer Einfülltrichter (51) aufgesetzt ist, der an eine Erdung angeschlossen und nach dem Füllvorgang verschließbar ist.

7. Vorrichtung nach Anspruch 6, dadurch gekennzeichnet, daß der Einfülltrichter (51) mit einer Flammensperre versehen ist.

8. Vorrichtung nach einem der Ansprüche 1 bis 5 zur Entsorgung von wässrigen anorganischen Lösungen, dadurch gekennzeichnet, daß der Aufnahmebehälter (50) aus einem gegenüber den zu entsorgenden Lösungen beständigen Kunststoffbehälter besteht, auf den ein Einfülltrichter (51) aufgesetzt ist, der mit einer Belüftung versehen ist.

9. Vorrichtung nach Anspruch 8, dadurch gekennzeichnet, daß der Einfülltrichter (51) aus einem trichterförmigen Bauteil besteht, an dessen Hals ein Einfüllstutzen und ein Überwurfdeckel angebracht ist, in dem Belüftungsöffnungen vorgesehen sind.

10. Vorrichtung nach Anspruch 9, dadurch gekennzeichnet, daß im Einfüllstutzen vertikal verschiebbar ein Peilstab angeordnet ist, der mit einem Schwimmer verbunden ist.

11. Vorrichtung nach Anspruch 9 oder 10, dadurch gekennzeichnet, daß im trichterförmigen Bauteil ein Sieb angeordnet ist.

12. Vorrichtung nach Anspruch 6 oder 8, dadurch gekennzeichnet, daß der Innenbehälter (40) eine derartige Höhe hat, daß seine Oberkante über der Oberkante des Einfülltrichters (51) liegt.

13. Vorrichtung nach einem der Ansprüche 1 bis 5 zur Entsorgung von Feststoffen, insbesondere chemisch benetzten Feststoffen, dadurch gekennzeichnet, daß als Aufnahmebehälter (50) ein Kombi-Behälter aus einem äußeren Stahlbehälter und einem Kunststoff-Weithals-Einstellbehälter vorgesehen ist.

## Claims

1. Device for the disposal of laboratory waste which comprises
- an outer body (20) which can be opened on one side and has, in the interior, a floor shelf (30) which can be pulled out from the opened side,
- an open-topped inner container (40) which is disposed removably on the floor shelf of the outer body (20) and has an adjustable exhaust ventilation system which can be connected to a ventilation plant,
- an insert tray (41) which is disposed on the floor of the inner container (40), and
- a container (50) for receiving the laboratory waste, held by the insert tray.

2. Device according to Claim 1, characterized in that one side of the inner container (40) can be opened and is connected, in the form of a double front, to the side of the outer body (20) which can be opened, both parts being able to pivot away for emptying after the floor shelf has been pulled out.

3. Device according to Claim 1 or Claim 2, characterized in that the insert tray (41) is a grating on which the receiving container (40) stands.

4. Device according to Claim 3, characterized by a transfer truck the transportation surface of which lies below the level of the grating in the insert tray (41).

5. Device according to any one of the preceding claims, characterized by a device (60-63) which opens a ventilation connector (60) when the floor shelf in the outer body (20) is pulled out and reduces the cross-sectional area of the ventilation connector (60) when the floor shelf is pushed back.

6. Device according to any one of the preceding claims for the disposal of solvents and combustible materials containing solvents, characterized in that the receiving container (50) is composed of a plastics container which is surrounded by a steel jacket and on which a metal funnel (51), connected to an earth connection and closable after filling, is placed.

7. Device according to Claim 6, characterized in that the funnel (51) has a flame trap.

8. Device according to any one of Claims 1 to 5 for the disposal of agueous inorganic solutions, characterized in that the receiving container (50) is composed of a plastics container which is resistant to the solutions to be disposed of and on which a funnel (51) provided with exhaust ventilation is placed.

9. Device according to claim 8, characterized in that the funnel (51) is constituted by a funnel-shaped component on the neck of which are mounted an inlet piece and a screw-cap union in which the exhaust ventilation openings are provided.

10. Device according to Claim 9, characterized in that a dipstick connected to a float is arranged so as to be movable vertically in the filler pipe.

11. Device according to Claim 9 or Claim 10, characterized in that a strainer is disposed in the funnel-shaped component.

12. Device according to Claim 6 or Claim 8, characterized in that the inner container (40) is of a height such that its upper edge lies above the upper edge of the funnel (51).

13. Device according to any one of Claims 1 to 5 for the disposal of solid matter, particularly chemically wetted solid matter, characterized in that a combination container of an outer steel container and a wide-necked plastics insert container is provided as the receiving container (50).

## Revendications

1. Dispositif pour l'enlèvement de déchets de laboratoire, comportant :
- un corps extérieur (20) qui peut être ouvert sur un côté et comprend à l'intérieur un panneau de fond (30) qui peut être extrait à partir de l'extérieur,
- un récipient intérieur (40) ouvert sur le côté supérieur qui est disposé sur le panneau de fond du corps extérieur (20) de manière à pouvoir être retiré et est pourvu d'un système d'évacuation d'air réglable qui peut être relié à une installation de ventilation,
- une cuvette (41) de garnissage qui est disposée sur le fond du récipient intérieur (40) et
- un récipient (50) de réception des déchets de laboratoire qui est maintenu par la cuvette (41).

2. Dispositif selon la revendication 1, caractérisé en ce qu'un côté du récipient intérieur (40) peut être ouvert et est en liaison avec le côté du corps extérieur (20) qui peut être ouvert, sous la forme d'une partie avant doublée, dont les deux éléments peuvent pivoter en s'écartant pour le vidage après extraction du panneau de fond.

3. Dispositif selon la revendication 1 ou 2, caractérisé en ce que dans la cuvette de garnissage (41) un caillebotis est disposé et supporte le récipient de réception (50).

4. Dispositif selon la revendication 3, caractérisé en ce qu'il comprend un chariot de transport dont la surface de transport est située dans la cuvette de garnissage (41) au-dessous de la hauteur du caillebotis.

5. Dispositif selon l'une quelconque des revendications précédentes, caractérisé par un agencement (60-63) qui débouche sur un tuyau d'aération (60) lorsque le panneau de fond du corps intérieur (20) est extrait, tandis que par coulissement en retour du panneau de fond la surface de la section transversale du tuyau d'aération (60) est réduite.

6. Dispositif selon l'une quelconque des revendications précédentes, pour l'enlèvement de détergents et de matière combustible contenant des détergents, caractérisé en ce que le récipient de réception (50) est constitué d'un récipient en matière plastique entouré d'une enveloppe d'acier et sur lequel est disposé un entonnoir métallique (51) de remplissage, qui est relié à la terre et peut être obturé après remplissage complet.

7. Dispositif selon la revendication 6, caractérisé en ce que l'entonnoir de remplissage (51) est pourvu d'un dispositif anti-retour de flammes.

8. Dispositif selon l'une des revendications 1 à 5 pour l'enlèvement de solutions aqueuses inorganiques, caractérisé en ce que le récipient de réception (50) est constitué d'un récipient en matière synthétique résistant aux solutions à évacuer et sur lequel est monté un entonnoir de remplissage (51) pourvu d'une aération.

9. Dispositif selon la revendication 8, caractérisé en ce que l'entonnoir de remplissage (51) est constitué par une pièce en forme d'entonnoir sur le col de laquelle est rapporté un tuyau de remplissage ainsi qu'un couvercle, des ouvertures d'aération étant prévues dans cette pièce en forme d'entonnoir.

10. Dispositif selon la revendication 9, caractérisé en ce qu'une jauge de niveau est montée coulissante verticalement dans le tuyau de remplissage, et est reliée à un flotteur.

11. Dispositif selon la revendication 9 ou 10, caractérisé en ce qu'un filtre est disposé dans la pièce en forme d'entonnoir.

12. Dispositif selon l'une des revendications 6 et 8, caractérisé en ce que le récipient interne (40) a une hauteur telle que son arête supérieure se trouve au-dessus de l'arête supérieure de l'entonnoir de remplissage (51).

13. Dispositif selon l'une quelconque des revendications 1 à 5 pour l'enlèvement de matières solides, notamment de matières solides humidifiées chimiquement, caractérisé en ce que comme récipient de réception (50) il est prévu un récipient combiné constitué d'un récipient extérieur en acier et d'un récipient de réglage en matière synthétique à col de grand diamètre.
